# EUROPÄISCHE PATENTSCHRIFT

(11) **EP 2 943 171 B1**
(45) Veröffentlichungstag und Bekanntmachung des Hinweises auf die Patenterteilung: **13.12.2023**
(21) Anmeldenummer: 14700195.2
(22) Anmeldetag: 09.01.2014
(51) Int. Cl.: A61F 13/02, A61F 13/00

(54) **FLÜSSIGKEITSDURCHLÄSSIGER PRIMÄRVERBAND MIT EINER SILIKONBESCHICHTUNG**
LIQUID-PERMEABLE PRIMARY DRESSING WITH A SILICONE COATING
PANSEMENT PRIMAIRE PERMÉABLE AUX LIQUIDES ET REVÊTU DE SILICONE

(30) Priorität: 09.01.2013 DE 102013100157
(43) Veröffentlichungstag der Anmeldung: 18.11.2015
(73) Patentinhaber: BSN medical GmbH, 22761 Hamburg (DE)
(72) Erfinder: Riesinger, Birgit, 48149 Münster (DE)
(74) Vertreter: Cohausz & Florack
(86) Internationale Anmeldenummer: PCT/EP2014/050331
(87) Internationale Veröffentlichungsnummer: WO 2014/108476

(56) Entgegenhaltungen:
- WO-A1-93/19710
- WO-A1-2007/118652
- WO-A1-2012/104584
- WO-A1-2012/140441
- WO-A1-2013/121006
- US-A- 5 340 363
- US-A1- 2012 095 380

## Beschreibung

Die vorliegende Erfindung betrifft einen Primärverband gemäß dem Oberbegriff des Anspruchs 1.

Primärverbände sind aus der Technik bekannt. Sie werden als Wundkontaktschicht auf zu behandelnde Wunden gelegt, bevor die eigentliche Wundauflage aufgelegt wird. Sie können verschiedenartige Eigenschaften aufweisen, beispielswiese die Haftung vermindern, das Einwachsen in die Wundauflage verhindern oder zu einem ausgewogenen Flüssigkeitsmanagement beitragen.

In anderen Ausführungsformen können Primärverbände als integraler Bestandteil der Hülle einer Wundauflage vorgesehen sein, wobei sie dort ähnliche oder identische Funktionen ausüben wie in frei vorliegender Form.

Im Prinzip ermöglichen Primärverbände auch eine Verlängerung der Verweildauer der eigentlichen Wundauflage auf der Wunde, was die Häufigkeit des Verbandswechsels vermindert und so traumatische Ereignisse reduziert. Allerdings sind viele der Wundtypen, bei denen eine Verwendung von Primärverbänden angezeigt ist (Dekubitus, Ulcus Cruris, Verbrennungswunden, etc.) höchst empfindlich gegenüber Verklebungen, und reagieren traumatisch auf Verbandswechsel, was für den Patienten äußerst schmerzhaft ist und ferner die Wundheilung stark beeinträchtigt.

Die WO 2013/121006 A1 betrifft einen flüssigkeitsdurchlässigen Wundverband und offenbart hierbei auch, dass der Primärverband ein mit Silikon beschichtetes Material aufweisen kann.

Die WO 2007/118652 A1 betrifft einen Primärverband und lehrt hierzu einen flächenförmigen, flüssigkeitsdurchlässigen Primärverband mit Perforationen (s. S. 2, zweiter bis vierter Absatz).

Aufgabe der vorliegenden Erfindung ist es daher, einen Primärverband bereitzustellen, der eine längere Verweildauer auf der Wunde ermöglicht.

Weitere Aufgabe der vorliegenden Erfindung ist es, einen Primärverband bereitzustellen, der einen atraumatischen Verbandswechsel ermöglicht.

Diese Aufgaben werden mit den Merkmalen des Anspruchs 1 gelöst. Die Unteransprüche geben bevorzugte Ausführungsformen an.

Demnach ist ein Flüssigkeitsdurchlässiger Primärverband in Bahnenform vorgesehen, aufweisend Poren und/oder Perforationen, die einen Flüssigkeitsdurchtritt ermöglichen, ferner aufweisend eine Beschichtung aus einem Silikonhaltigen Material.

Solche Primärverbände werden als Wundkontaktschicht auf zu behandelnde Wunden gelegt, bevor die eigentliche Wundauflage aufgelegt wird. Sie können verschiedenartige Eigenschaften aufweisen, beispielswiese die Haftung vermindern oder zu einem ausgewogenen Flüssigkeitsmanagement beitragen.

Der Begriff "Silikonhaltiges Material" wie hier verwendet, betrifft sowohl silikonbeschichtetes Material als auch aus Vollsilikon bestehendes Material.

Das Silikon kann so ausgebildet sein, dass es dezidiert adhäsiv wirkt, dergestalt, dass der Primärverband an der Wunde haftet, ebenso aber auch dergestalt, dass er eine Haftung des ggf. häufig zu wechselnden Sekundärverbandes an der Wunde, oder gar eine Eingranulation desselben, verhindert.

Die Haftungseigenschaften von Silikon können technisch sehr genau eingestellt werden, sodass (a) ein sicheres Haften gewährleistet werden kann, ohne dass ein Ablösen des Primärverbandes Schmerzen - etwa durch anhaftende Körperbehaarung - erzeugt oder gar traumatisch wirkt, oder (b) gewährleistet werden kann, dass der Primärverband nicht haftet. Erfindungsgemäß ist dabei vorgesehen, dass die Beschichtung linienförmig, streifenförmig, punktförmig und/oder tropfenförmig ausgebildet ist. Hierzu wird auf die Figuren verwiesen. Erfindungsgemäß ist ferner vorgesehen, dass der Primärverband eine Folie aufweisend Poren und/oder Perforationen aufweist. Besagte Folie besteht bevorzugt aus einer thermoplastischen Folie oder aus einem Polyolefin, beispielsweise PE. Solche Folien werden beispielsweise von den Firmen Tredegar oder RKW hergestellt.

Die Silikonbeschichtung kann dabei vor Einarbeitung der Poren und/oder Perforationen auf die Folie aufgebracht sein, oder aber erst nach Einarbeitung der Poren und/oder Perforationen.

Erfindungsgemäß ist ferner vorgesehen, dass die Poren und/oder Perforationen dreidimensional ausgestaltet sind.

Bevorzugt ist ferner vorgesehen, dass der Primärverband eine rauhe und eine glatte Seite aufweist. Auf diese Art und Weise entsteht ein Material ähnlich dem Produkt Sorbion Plus. Dieses ist in der Patentanmeldung WO2007118652 beschrieben.

Bei Applikation der glatten Seite auf die Wunde kommen neben den üblichen Vorteilen von Primärverbänden (geringe Haftung an die Wunde, Verhinderung der Eingranulation von Wundmaterial in die Wundauflage) auch eine flüssigkeitsleitende Funktion zum Tragen; so werden durch die dreidimensionalen Poren und/oder Perforationen Kragen ausgebildet, die auf der Wundabgewandten Seite angeordnet sind und einen Rückfluss von Wundexsudat in die Wunde behindern.

Bei Applikation der rauhen Seite auf die Wunde entwickelt der Primärverband u.U. eine abrasive Wirkung, die in der Lage ist, Biofilme aufzubrechen und ein Debridement herbeizuführen. Diese Wirkung ist in der Patentanmeldung DE 102012100842 beschrieben. Ferner ist dabei bevorzugt vorgesehen, dass die Beschichtung auf der rauhen und/oder auf der glatten Seite angeordnet ist - je nachdem welche Seite als Wundkontaktseite zum Einsatz kommen soll.

Als ein Aspekt der nicht erfindungsgemäß ist und lediglich der Veranschaulichung dient, ist vorgesehen, dass der Primärverband eine Gaze oder ein Gewebe aufweisend Poren und/oder Gitterwaben aufweist. Solche Produkte sind beispielsweise unter den Markennamen Adaptic (bestehend aus einem Cellulose-Acetat-Netz) bekannt.

Ferner ist vorgesehen, dass der Primärverband aufweisend einen Anteil an mindestens einem elementaren oder in Ionenform vorliegenden Schwermetall aufweist.

Schwermetalle wirken in feinstverteilter Form bakterizid, was aufgrund der großen reaktiven Oberfläche auf die hinreichende Entstehung von löslichen Schwermetallionen zurückzuführen ist.

Durch die Dotierung mit mindestens einem elementaren oder in Ionenform vorliegenden Schwermetall kann dem primärverband eine antibakterielle Wirkung verliehen werden, was Komplikationen bei infektionsgefährdeten Wunden (Dekubitus, Ulcus Cruris, Verbrennungswunden, etc.) vermindert und gleichzeitig die Verweildauer der Wundauflage erhöhen kann.

Bevorzugt ist vorgesehen, dass das mindestens eine elementare oder in Ionenform vorliegenden Schwermetalle ausgewählt ist aus der Gruppe enthaltend Kupfer, Zink und/oder Silber. Die oben genannten bakteriziden Eigenschaften gelten in besonderer Form für diese drei Metalle.

Bevorzugt ist ferner vorgesehen, dass die elementaren oder in Ionenform vorliegenden Schwermetalle durch Beschichten auf den Primärverband aufgebracht sind. Bevorzugte Beschichtungsverfahren sind beispielsweise:
- Chemische Gasphasenabscheidung (CVD)
- Flammenbeschichtung (C-CVD)
- Physikalische Gasphasenabscheidung (PVD)
- Plasmagestützte Chemische Gasphasenabscheidung (PECVD)
- Rotationsbeschichtung (spin coating)
- Spritzen
- Tauchbeschichten (Dipcoating)
- Vakuumverdampfen
- Sputtern

Dem Fachmann sind die gängigen Methoden bekannt, und er ist daher in der Lage, ohne weitere erfinderische Tätigkeit die oben genannten Verfahren zum Beschichten des erfindungsgemäßen Primärverbandes mit elementaren oder in Ionenform vorliegenden Schwermetallen anzuwenden.

Bevorzugt ist ferner vorgesehen, dass die elementaren oder in Ionenform vorliegenden Schwermetalle durch Coextrusion in den Primärverband eingebracht sind. Hierbei können die elementaren oder in Ionenform vorliegenden Schermetalle beispielsweise in Form von Kolloiden, in Form von Salzen (bevorzugt als Chlorid, Sulfat oder Nitrat) oder in Form von Metallorganischen Verbindungen in den Extrusionsprozess eingebracht werden.

Ebenso kann die Beschichtung oder das Material für die Extrusion Dotierung aus einer Kombination aus Silber, Zink oder Kupfer und Calciumphosphat (beispielsweise in Form von Nanopartikeln aus Calciumphosphat, die mit Silber, Zink oder Kupfer beschichtet sind) bestehen. Die Kombination eines der drei Schwermetalle mit Calciumphosphat ist für viele Keime bis zu 1000 Mal tödlicher als herkömmliche Silber-Präparate. Ein entscheidender Faktor scheint zu sein, dass Bakterien den Trägerstoff Calcium für ihren Stoffwechsel nutzen. Die 20 bis 50 Nanometer grossen Calciumphosphat-Partikel werden von den Mikroorganismen als Nahrung aufgenommen und dadurch zersetzt. Dabei werden tausende von 1 bis 2 Nanometer kleinen Silberpartikeln, freigegeben, und entfalten ihre bakteriostatische Wirkung.

Bevorzugt ist ferner vorgesehen, dass es sich bei dem Primärverband um ein Wunddistanzgitter handelt.

Ferner ist vorgesehen, dass der Primärverband darüber hinaus Stanzungen, Schlitze, Inzisionen und/oder Ausnehmungen aufweist, die einem erleichterten Flüssigkeitsdurchtritt dienen.

Diese können beispielsweise in Form von Langlöchern, Karos und/oder oder Kreuzen ausgebildet sein. Dies ist vorteilhaft, wenn das Material aus einem dreidimensionalen Folienmaterial mit nach außen bzw. zur Wunde gekehrten Öffnungen bzw. Perforationen besteht, die dem Wundpflegeartikel eine rauhe Außenfläche und damit abrasive Eigenschaften verleihen. Durch die abrasiven Eigenschaften wird die Exsudation der Wunde angeregt, und es kann zu Flüssigkeitsansammlungen im oberen Wundbereich kommen, die abgeleitet werden müssen. Die genannten Stanzungen, Schlitze, Inzisionen und/oder Ausnehmungen gewährleisten einen erleichterten Durchtritt sowie eine effektive und schnelle Aufnahme des Exsudats, dass durch Verwendung des erfindungsgemäßen Primärverbands erzeugt wird.

Ferner ist eine Wundauflage vorgesehen, aufweisend eine Hülle die mindestens abschnittsweise aus einem erfindungsgemäßen Primärverband besteht.

Solche Konfigurationen, bei denen ein Primärverband in Bahnenform, aufweisend Poren und/oder Perforationen den Teil der Hülle einer Wundauflage ausbildet, finden in der Wundversorgung häufig Anwendung. So weisen beispielsweise die Produkte Curea P2 und Vliewasorb einen solchen Primärverband - freilich ohne die erwähnten Schwermetalle - als integralen Bestandteil Ihrer Hülle auf.

Bevorzugt ist vorgesehen, dass besagte Wundauflage einen Wundexsudate absorbierenden Körper aufweist. Bevorzugt weist der Wundexsudate absorbierende Körper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und/oder eine lose Füllung aus superabsorbierenden Polymeren. Besagte Airlaidmatte kann bevorzugt einen im Wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Wundexsudate absorbierende Körper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion Sachet" vertrieben wird

Der Wundexsudate absorbierende Körper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt.

Für den absorbierenden Körper kommen ebenso die bereits zuvor genannten Mikrofasern in Frage. Diese können sowohl alleine als auch in Kombination mit anderen Fasern sowie den superabsorbierenden Polymeren verwendet werden.

Der Wundexsudate absorbierende Körper kann in einer anderen Ausgestaltung ebenso mindestens eine flache Lage aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren lagen können dabei in einer bevorzugten Ausgestaltung auch durch walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

In einer besonderen Ausführungsform ist vorgesehen, dass der Wundpflegeartikel, insbesondere der Wundexsudate absorbierende Körper, superabsorbierende Polymere aufweist. Diese können bei einer Wundauflage in die absorbierenden Körper und/oder in die Hülle eingearbeitet sein, oder aber als loser Bestandteil innerhalb der Hülle platziert werden. Letztere Anordnung bedingt eine der Größe der superabsorbierenden Polymere angepasste Hülle, bei der die superabsorbierenden Polymere nicht aus der Hülle hinaus rieseln. Wie noch weiter ausgeführt wird, sind sowohl superabsorbierende Partikel oder aber Fasern möglich, die entweder als lose Schüttung vorliegen oder aber in das umgebende Material eingearbeitet sind. Auch bei einem Wundreinigungspad ist die Fixierung der superabsorbierenden Polymere der Ausgestaltung des Pads oder Tuches anzupassen.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in das Polymerpartikel quillt es auf und strafft auf molekularer Ebene dieses Netzwerk, so dass das Wasser ohne Hilfe nicht mehr entweichen kann.

Die Verwendung von SAP in Wundauflagen zur Aufnahme von Exsudat ist bereits aus der WO0152780 und der WO03094813 der Anmelderin der vorliegenden Erfindung bekannt. Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert sind und sowohl bestehendes als auch neu gebildetes Collagen in der Wunde abbauen.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer Regression der Wundheilung beiträgt. Das pathologische Exsudat ist hinsichtlich des Gehalts seiner Komponenten aus dem der Wundprogression förderlichem Gleichgewicht geraten. Daraus ergeben sich verschiedene Komplikationen, die zur weiteren Verschlechterung und Chronifizierung der Wunde beitragen.

Im Zusammenhang mit dem erfindungsgemäßen Wundpflegeartikel aufweisend mindestens eine Oberfläche mit abrasiven Eigenschaften, der imstande ist, in der Wunde angeordnete Biofilme aufzubrechen, und/oder die Wundexsudation anzuregen, kommt der besagten Komponente aufweisend SAP eine besondere Bedeutung zu.

So dient der vorgesehene Anteil an SAP dazu, die erzeugten Bruchstücke und Überreste des Biofilms aufzunehmen. Durch die Zerstörung des Biofilms werden ggf. auch Endotoxine und bakterielle Pathogenitätsfaktoren (insbesondere bakterielles Hämolysin und Leukocidin) frei- gegeben, die beim Patienten Entzündungen, Allergien, Schocks (insbesondere anaphylaktische Schocks und/oder das Toxic Shock Syndrome) und Fieber erzeugen können (Herxheimer-Reaktion). Besagte Endotoxine und Pathogenitätsfaktoren werden durch den Anteil an SAP aufgenommen, so dass die besagten Folgen vermieden können.

In der DE102007054127 der Anmelderin der vorliegenden Erfindung wird auf diese Verhältnisse genau eingegangen; insbesondere wird gezeigt, dass SAP in der Lage sind, Bakterien und bakterielle Endotoxine zu binden.

Dabei ist besonders bevorzugt vorgesehen, dass der Wundexsudate absorbierende Körper ein Muster aus Inzisionen und/oder Stanzungen aufweist. Diese sind bevorzugt dergestalt ausgebildet und/oder angeordnet, dass sie den Flüssigkeitseintritt in den Wundpflegeartikel erleichtern.

Dieses Merkmal entfaltet besondere Vorteile im Zusammenspiel mit einer Hülle, die dem Wundpflegeartikel eine rauhe Außenfläche und damit abrasive Eigenschaften verleihen, beispielsweise aus einem dreidimensionalen Folienmaterial mit nach außen bzw. zur Wunde gekehrten Öffnungen bzw. Perforationen, da so eine effektive und schnelle Aufnahme des Exsudats, dass durch Verwendung des erfindungsgemäßen Wundpflegeartikels erzeugt wird, zu gewährleisten. Ferner ist ein Primärverband oder eine Wundauflage gemäß der Erfindung zur Verwendung in einem Verfahren zur Wundbehandlung durch Verwendung in einem Unterdruck-Wundversorgungssystem vorgesehen.

Solche Systeme sind z.B. in den Schriften DE202004017052, WO2006048246 und DE202004018245 der Anmelderin der vorliegenden Erfindung offenbart.

Aus erstgenannter ist eine Vorrichtung zur Wundbehandlung unter Einsatz von Unterdruck bekannt, aufweisend ein gasdichtes Wundabdeckungselement, das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einer flächenhaften, die Wundexsudate aufnehmenden Wundauflage unterlegt ist, dessen Volumen im Laufe des Absorptionsprozesses zunimmt, so dass die absorbierten Wundexsudate innerhalb der Wundauflage und damit unterhalb des Wundabdeckungselementes bis zur Entfernung der Wundauflage aus dem Körper des Patienten verbleiben, die Wundauflage wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich die Wundauflage in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend: ein Wundabdeckungselement zur Anbringung an Haut- und Schleimhautoberfläche, wenigstens eine Anschlussstelle, die mit dem Wundraum in Kontakt steht und über welche die im Wundraum befindlichen Stoffe evakuiert werden können, wobei dieser superabsorbierende Polymere aufweist, wobei die absorbierten Wundexsudate an Polymere gebunden im Wundraum bis zur Entfernung aus dem Wundraum verbleiben, wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens eine innerhalb des Raumes angeordnete, die Wundexsudate aufsaugende Wundauflage, die wenigstens eine Lage eines mit Superabsorbentien angereicherten Textilabschnittes aufweist, die mit einer flüssigkeitsdurchlässigen Hülle umgeben ist, wobei die absorbierten Wundexsudate innerhalb der Wundauflage und damit unterhalb des Wundabdeckungselementes bis zur Entfernung der Wundauflage aus dem Körper des Patienten verbleiben, und wobei das Wundabdeckungselement eine gasdicht verschliessbare Behandlungsöffnung aufweist, durch die die Wundauflage in den Raum einlegbar und aus dem Raum entnehmbar ist.

### Zeichnungen

Die Erfindung ist in den beigefügten Zeichnungen in nicht einschränkender Weise exemplarisch dargestellt.

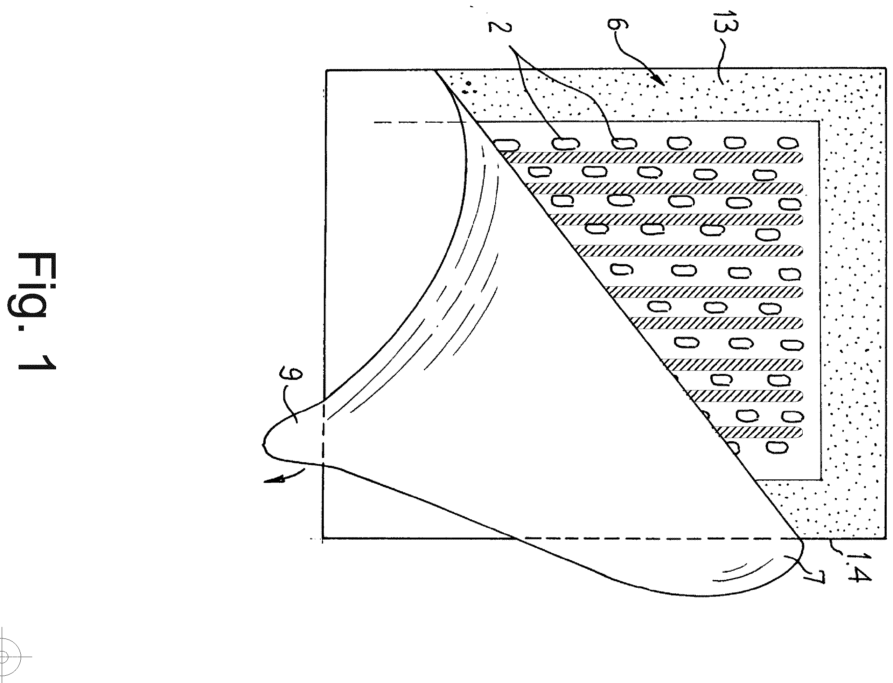

Fig. 1 zeigt einen Flüssigkeitsdurchlässigen Primärverband in Bahnenform, aufweisend Perforationen 2, die einen Flüssigkeitsdurchtritt ermöglichen. Der Primärverband ist in einer sterilen Verpackung 9, 14 untergebracht. Der Primärverband weist eine flächige Silikonbeschichtung auf. Ferner zeigt Fig. 1 eine Silikonbeschichtung in Form von streifenförmig auf den primärverband aufgebrachten Silikonbändern.

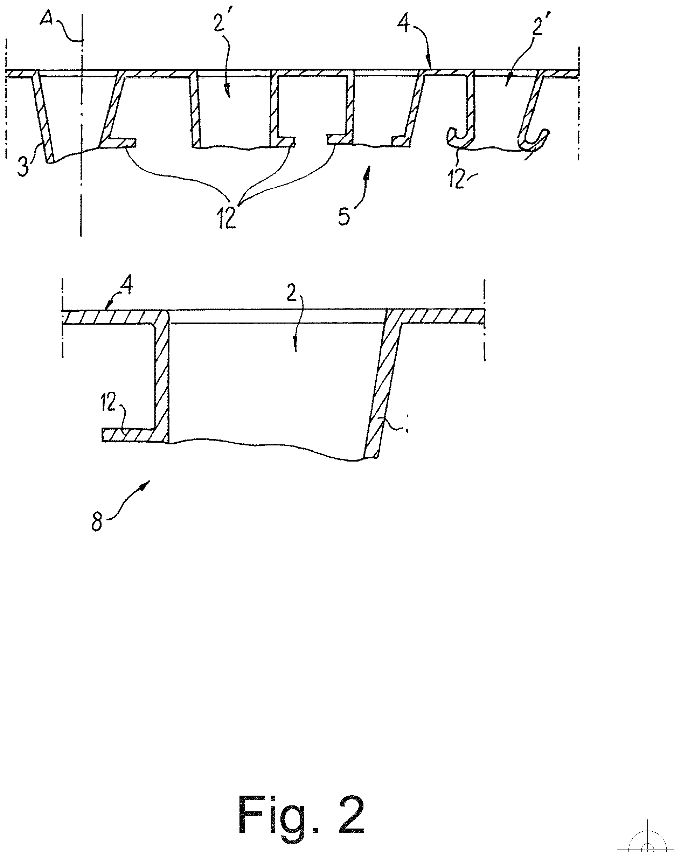

Fig. 2 zeigt besagte Perforationen im Querschnitt. Dabei ist erkennbar, dass letztere bevorzugt konisch ausgebildete Wandungen besitzen, welche wiederum unregelmässig in etwa senkrecht zu einer Perforationsachse A gerichtete Auskragungen auslaufen. Diese Auskragungen können auch nach innen oder nach aussen umgekrempelt sein, wie es die rechte Seite der Fig. 2 zeigt. Der beschriebene Aufbau der Perforationen trägt dazu bei, dass das aufgenommene Wundexsudat nur erschwert in Richtung der Wunde zurückfliessen kann, dabei sind besagte Auskragungen nicht unbedingt erforderlich.

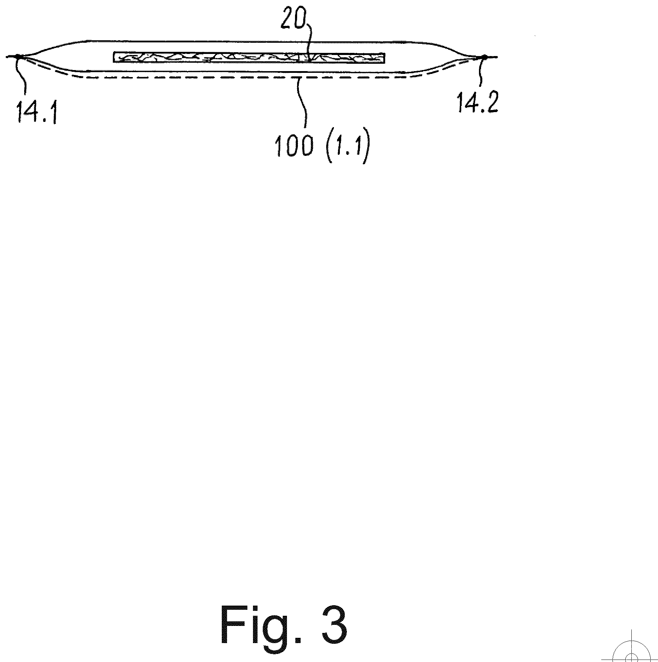

Fig. 3 zeigt eine Wundauflage mit einem flächigen Absorptionskörper und einer Hülle, die abschnittsweise aus einem Primärverband gemäß einem der vorherigen Patentansprüche besteht gestrichelte Linie). Besagter Primärverband kann an der betreffenden Seite die eigentliche Hüllwand ergänzen (doppeln) oder auch ersetzen (nicht dargestellt). Besagte Wundauflage weist darüber hinaus einen Wundexsudate absorbierenden Körper auf.

Abweichend von der Darstellung in Fig. 3 kann der Primärverband den Absorptionskörper auch allseitig umgeben, dabei die Hülle ergänzen oder aber ersetzen. Die Nähte (beispielsweise Ultraschallnähte) können abweichend von der Darstellung auch nach innen gekehrt sein (die Hülle also quasi "auf links gezogen" vorliegen), um weiche und für den Wundkontakt angenehme Kanten auszubilden.

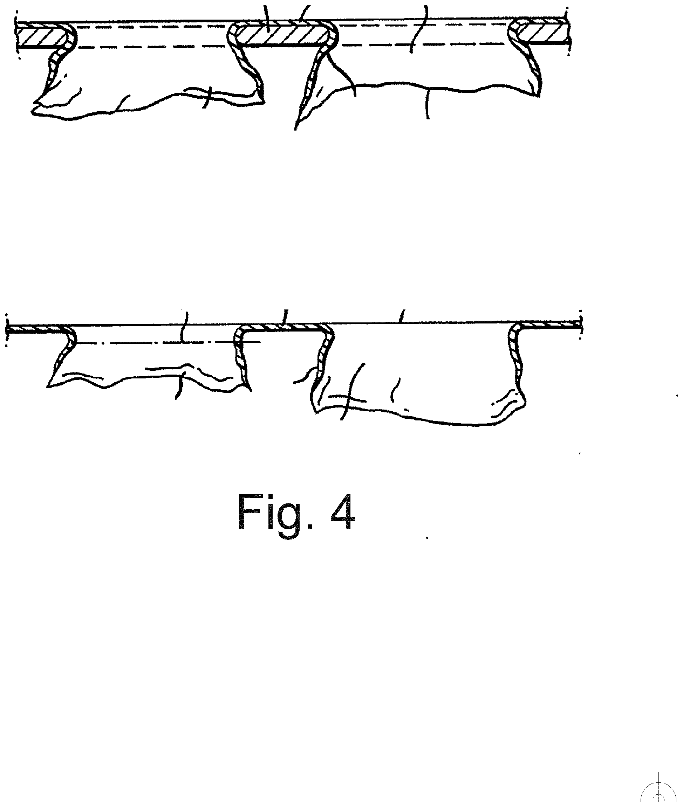

Fig. 4 zeigt ebenfalls die besprochenen Perforationen im Querschnitt, diesmal in einer naturalistischen Darstellung. Hier ist zu erkennen, dass die in Fig. 2 beschriebenen Auskragungen nicht vorkommen.

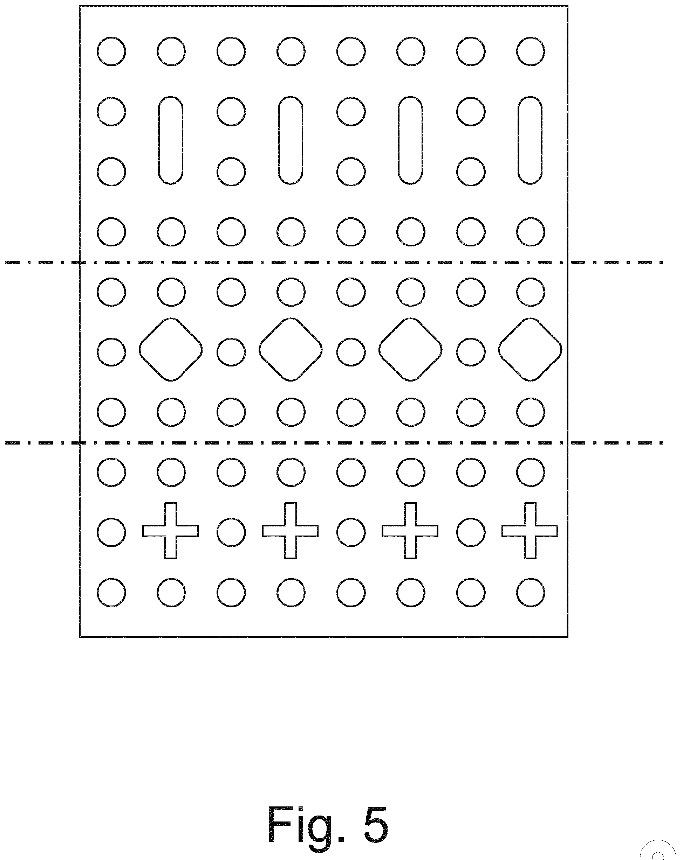

Fig. 5 zeigt einen ähnlichen Flüssigkeitsdurchlässigen Primärverband in Bahnenform wie Fig. 1, aufweisend Perforationen. Auch hier öffnen sich die Perforationen zur Wundseite hin und bilden so die besprochene rauhe, abrasiv wirkende Oberfläche aus. Der Flüssigkeitsdurchlässige Primärverband in Bahnenform kann teils oder vollständig als Hülle für einen Wundpflegeartikel aufweisend einen absorbierenden Körper agieren. Ferner weist dieser Wundpflegeartikel Stanzungen, Schlitze, Inzisionen und/oder Ausnehmungen beispielsweise in Form von Langlöchern, Karos und/oder oder Kreuzen auf, die einem erleichterten Flüssigkeitsdurchtritt dienen.

Dies ist besonders dann vorteilhaft, wenn das Material aus einem dreidimensionalen Folienmaterial mit nach außen bzw. zur Wunde gekehrten Öffnungen bzw. Perforationen besteht, die dem Wundpflegeartikel eine rauhe Außenfläche und damit abrasive Eigenschaften verleihen. Durch die abrasiven Eigenschaften wird die Exsudation der Wunde angeregt, und es kann zu Flüssigkeitsansammlungen im oberen Wundbereich kommen, die abgeleitet werden müssen. Die genannten Stanzungen, Schlitze, Inzisionen und/oder Ausnehmungen gewährleisten einen erleichterten Durchtritt sowie eine effektive und schnelle Aufnahme des Exsudats, dass durch Verwendung des erfindungsgemäßen Wundpflegeartikels erzeugt wird.

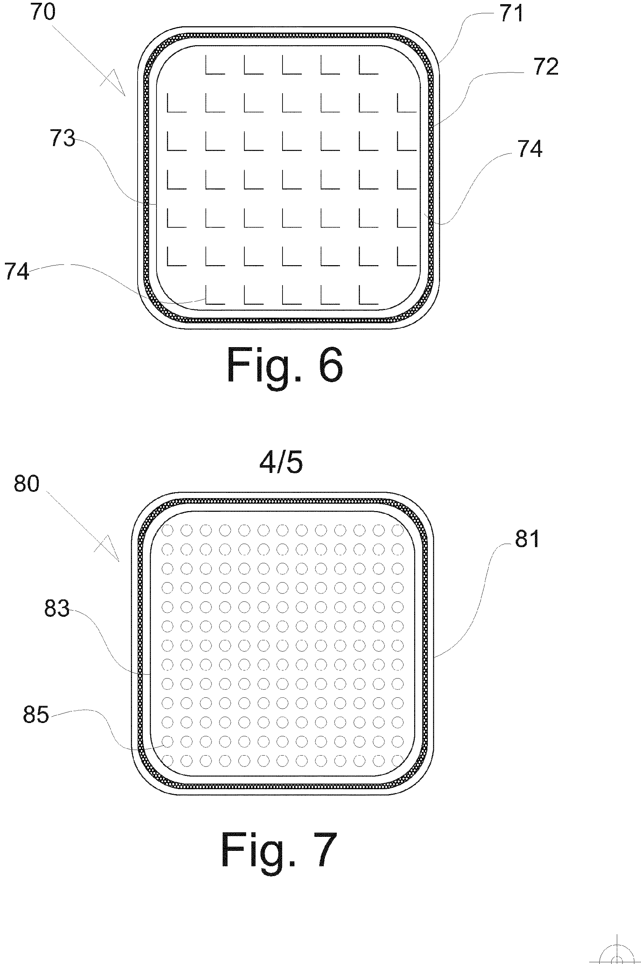

Fig. 6 zeigt einen Wundpflegeartikel 70 in Draufsicht, aufweisend eine Hülle 71 mit einer Naht 72, die aus einem dem erfindungsgemäßen Primärverband besteht, bevorzugt mit nach außen gekehrten Öffnungen bzw. Perforationen besteht, die dem Wundpflegeartikel eine rauhe Außenfläche und damit abrasive Eigenschaften verleihen, sowie einem flächigen Absorptionskörper 73 aus einem Vlies- oder Airlaidmaterial enthaltend superabsorbierende Polymere. Die Hülle bildet einen Expansionsraum 74 aus, sodass gewährleistet ist, dass der Absorptionskörper bei Flüssigkeitsaufnahme in seinem Volumen zunehmen kann und nicht durch die Hülle eingeschränkt ist. Der flächige Absorptionskörper 73 weist ein Muster aus L-Förmigen Inzisionen 75 auf, die mittels eines entsprechend ausgestalteten Stanzwerkzeugs in den Absorptionskörper eingebracht sind. Auf diese Weise wird der Flüssigkeitseintritt in den Wundpflegeartikel wesentlich erleichtert. Dieses Merkmal entfaltet besondere Vorteile im Zusammenspiel mit der Hülle aus einem dreidimensionalen Folienmaterial mit nach außen gekehrten Öffnungen bzw. Perforationen, die dem Wundpflegeartikel eine rauhe Außenfläche und damit abrasive Eigenschaften verleihen.

Abweichend von der Darstellung in Fig. 6 kann die Naht 72 (beispielsweise Ultraschallnähte) auch nach innen gekehrt sein (die Hülle also quasi "auf links gezogen" vorliegen), um weiche und für den Wundkontakt angenehme Kanten auszubilden.

Fig. 7 zeigt einen Wundpflegeartikel 80 in Draufsicht, aufweisend eine ähnlich wie in Fig. 1 gestaltete Hülle 81 aus dem erfindungsgemäßen Primärverband sowie einen flächigen Absorptionskörper 83 aus einem Vlies- oder Airlaidmaterial enthaltend superabsorbierende Polymere. Der flächige Absorptionskörper 83 weist ein flächiges Muster aus Lochstanzungen 85 auf, die mittels eines entsprechend ausgestalteten Stanzwerkzeugs in den Absorptionskörper eingebracht sind. Auf diese Weise wird der Flüssigkeitseintritt in den Wundpflegeartikel erleichtert. Ferner wird die Anschmiegsamkeit des ursprünglich relativ steifen Absorptionskörpers erhöht, so dass ein Wundpflegeartikel erzielt wird, der sich sanft an das Wundrelief anschmiegt, vom Patienten als sehr weich und angenehm empfunden wird und durch den engen Kontakt zur Wunde seine Wundexsudate aufnehmende Funktion voll entfalten kann.

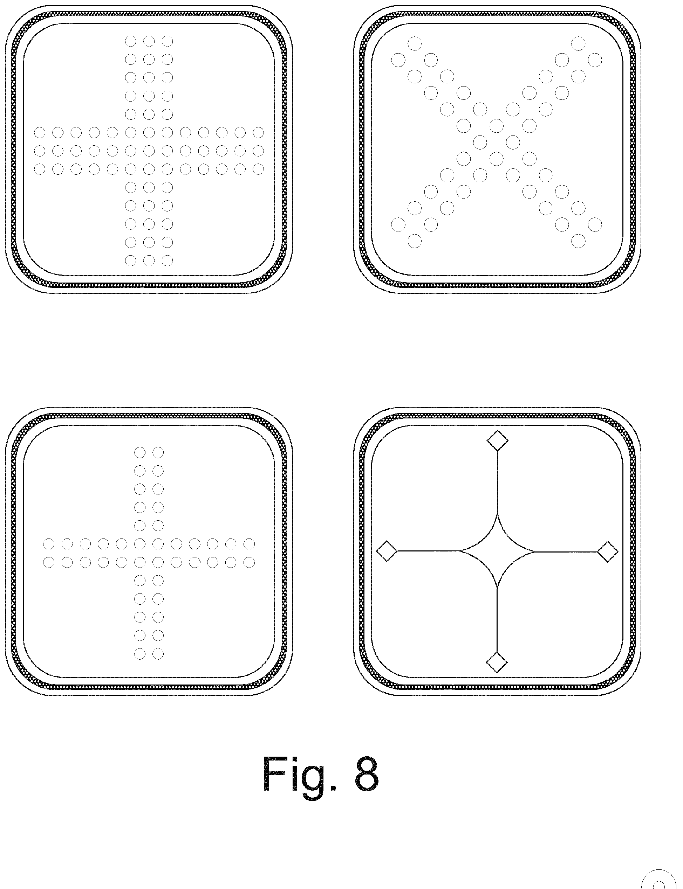

Fig. 8 zeigt weitere Ausgestaltungen des erfindungsgemäßen Wundpflegeartikels, wobei die Stanzungen und/oder Inzisionen, die teils gemeinsam in einem Wundpflegeartikel verwirklicht sind, den Flüssigkeitseintritt in den Wundpflegeartikel erleichtern.

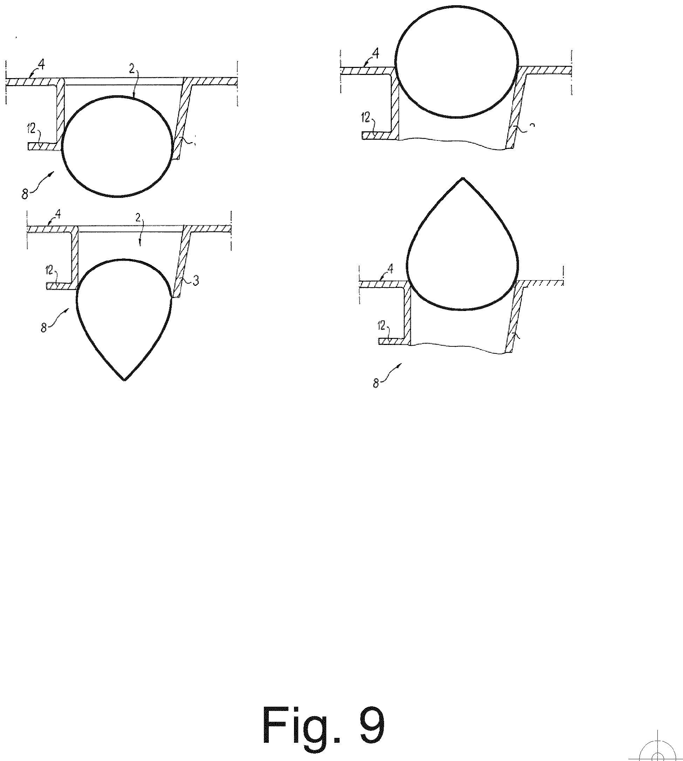

Fig. 9 zeigt nochmals die Perforationen im Querschnitt. Auf besagten Perforationen sind beispielhaft im Querschnitt punkt-, tropfen- oder linienförmig aufgebrachte Silikonbeschichtungen dargestellt.

## Patentansprüche

1. Flüssigkeitsdurchlässiger Primärverband in Bahnenform, aufweisend eine Folie aufweisend Poren und/oder Perforationen, die einen Flüssigkeitsdurchtritt ermöglichen, die Folie ferner aufweisend eine Beschichtung aus einem Silikonhaltigen Material, **dadurch gekennzeichnet, dass** die Beschichtung linienförmig, streifenförmig, punktförmig und/oder tropfenförmig ist;
wobei die Poren und/oder Perforationen dreidimensional ausgestaltet sind.

2. Primärverband gemäß einem der vorherigen Ansprüche, wobei der Primärverband eine rauhe und eine glatte Seite aufweist.

3. Primärverband gemäß Anspruch 2, wobei die Beschichtung auf der rauhen und/oder auf der glatten Seite angeordnet ist.

4. Primärverband gemäß einem der vorherigen Ansprüche, wobei es sich bei dem Primärverband um ein Wunddistanzgitter handelt.

5. Primärverband gemäß einem der vorherigen Ansprüche, wobei der Primärverband darüber hinaus Stanzungen, Schlitze, Inzisionen und/oder Ausnehmungen aufweist, die einem erleichterten Flüssigkeitsdurchtritt dienen.

6. Wundauflage, aufweisend eine Hülle, die mindestens abschnittsweise aus einem Primärverband gemäß einem der vorherigen Patentansprüche besteht.

7. Wundauflage, gemäß Anspruch 6, wobei besagte Wundauflage einen Wundexsudate absorbierenden Körper aufweist.

8. Wundauflage gemäß Anspruch 7, **dadurch gekennzeichnet, dass** der Wundexsudate absorbierende Körper ein Muster aus Inzisionen und/oder Stanzungen aufweist.

9. Wundauflage gemäß Anspruch 8, **dadurch gekennzeichnet, dass** die Inzisionen und/oder Stanzungen dergestalt ausgebildet und/oder angeordnet sind, dass sie den Flüssigkeitseintritt in die Wundauflage erleichtern.

10. Ein Primärverband oder eine Wundauflage gemäß einem der vorherigen Patentansprüche zur Verwendung in einem Verfahren zur Wundbehandlung durch Verwendung in einem Unterdruck-Wundversorgungssystem

## Claims

1. A fluid-permeable primary dressing in web form, comprising a film having pores and/or perforations which allow fluid to pass through, the film further comprising a coating made of a silicone-containing material, **characterized in that** the coating is line-shaped, strip-shaped, spot-shaped and/or drop-shaped;
the pores and/or perforations being three-dimensional.

2. The primary dressing according to any of the preceding claims, wherein the primary dressing has a rough and a smooth side.

3. The primary dressing according to claim 2, wherein the coating is arranged on the rough and/or on the smooth side.

4. The primary dressing according to any of the preceding claims, wherein the primary dressing is a wound spacer grid.

5. The primary dressing according to any of the preceding claims, wherein the primary dressing also has punched holes, slots, incisions and/or recesses which are used to facilitate fluid passing through.

6. A wound dressing comprising a covering which consists at least in portions of a primary dressing according to any of the preceding claims.

7. The wound dressing according to claim 6, wherein said wound dressing has a body that absorbs wound exudates.

8. The wound dressing according to claim 7, **characterized in that** the body that absorbs wound exudates has a pattern made of incisions and/or punched holes.

9. The wound dressing according to claim 8, **characterized in that** the incisions and/or punched holes are designed and/or arranged such that they facilitate fluid passing into the wound dressing.

10. A primary dressing or a wound dressing according to any of the preceding claims for use in a method for treating wounds by use in a vacuum wound care system.

## Revendications

1. Pansement primaire perméable aux liquides sous forme de bande, présentant un film présentant des pores et/ou des perforations qui permettent un passage de liquide, le film présentant en outre un revêtement en un matériau contenant du silicone, **caractérisé en ce que** le revêtement est en forme de ligne, de rayure, de point et/ou de goutte ;
dans lequel les pores et/ou les perforations sont conçus de manière à être tridimensionnels.

2. Pansement primaire selon l'une des revendications précédentes, dans lequel le pansement primaire présente un côté rugueux et un côté lisse.

3. Pansement primaire selon la revendication 2, dans lequel le revêtement est disposé sur le côté rugueux et/ou sur le côté lisse.

4. Pansement primaire selon l'une des revendications précédentes, dans lequel le pansement primaire est un moyen d'espacement pour la plaie.

5. Pansement primaire selon l'une des revendications précédentes, dans lequel le pansement primaire présente en outre des découpes, des fentes, des incisions et/ou des évidements qui servent à faciliter un passage de liquide.

6. Pansement pour plaie, présentant une enveloppe qui est constituée au moins dans certaines sections d'un pansement primaire selon l'une des revendications de brevet précédentes.

7. Pansement pour plaie selon la revendication 6, dans lequel ledit pansement pour plaie présente un corps absorbant l'exsudat de la plaie.

8. Pansement pour plaie selon la revendication 7, **caractérisé en ce que** le corps absorbant l'exsudat de la plaie présente un motif constitué d'incisions et/ou de découpes.

9. Pansement pour plaie selon la revendication 8, **caractérisé en ce que** les incisions et/ou les découpes sont réalisées et/ou disposées de manière à ce qu'elles facilitent l'entrée de liquide dans le pansement pour plaie.

10. Pansement primaire ou pansement pour plaie selon l'une des revendications de brevet précédentes destiné à être utilisé dans un procédé permettant le traitement de plaie par utilisation dans un système de soins de plaie par pression négative.
